# EUROPEAN PATENT APPLICATION

(11) **EP 4 218 861 A1**
(43) Date of publication of application: **02.08.2023**
(21) Application number: 21886256.3
(22) Date of filing: 27.10.2021
(51) Int. Cl.: A61M 5/31

(54) **SYRINGE**

(30) Priority: 27.10.2020 JP 2020179783
(71) Applicant: Taisei Kako Co., Ltd., Kita-ku Osaka-shi Osaka 531-0072 (JP); CHUGAI SEIYAKU KABUSHIKI KAISHA, Tokyo 115-8543 (JP)
(72) Inventor: YAMANAKA, Yuji, Tokyo 115-8543 (JP); EGAMI, Kiichi, Tokyo 115-8543 (JP); HORITA, Taiji, Ibaraki-shi, Osaka 567-0054 (JP); OGAWA, Yukihiro, Ibaraki-shi, Osaka 567-0054 (JP); TANIGUCHI, Kensuke, Ibaraki-shi, Osaka 567-0054 (JP); MIYAGAWA, Junki, Ibaraki-shi, Osaka 567-0054 (JP)
(74) Representative: DTS Patent- und Rechtsanwälte Schnekenbühl und Partner mbB
(86) International application number: PCT/JP2021/039614
(87) International publication number: WO 2022/092138

(57) **Abstract**

The present invention includes: a barrel formed into a cylindrical shape with a distal end part and a proximal end, the distal end part provided with an outlet opening for discharging to the outside the medicine contained inside the barrel therethrough; a filter disposed closer to a proximal side than the outlet opening inside the barrel; and a holding member configured to hold the filter and including a proximal side part disposed closer to the proximal side than the filter. The proximal side part of the holding member has a flow path through which the medicine flows, a proximal side space into which the medicine flows through the flow path is provided on the proximal side of the filter, a distal side space into which the medicine flows from the proximal side space via the filter is provided on the distal side of the filter, and an area of the outlet opening is smaller than an area of the proximal side space, smaller than an area of the distal side space, and smaller than an area of the flow path, as viewed from an axial direction of the barrel.

## Description

### CROSS-REFERENCE TO RELATED APPLICATION

This application claims priority to Japanese Patent Application No. 2020-179783, the disclosure of which is incorporated herein by reference in its entirety.

### FIELD

The present invention relates to a syringe including a filter capable of, for example, filtering out particles in a medicine at the time of injecting the medicine.

### BACKGROUND

Conventionally known as a syringe for injecting a medicine is a cylinder including a filter body (Patent Literature 1). A fixing member provided with an injection needle is detachably attached to a distal end of the cylinder. Further, a filter body for filtering out foreign substances and allowing only a medical liquid to flow therethrough is disposed within the distal end of the cylinder.

### CITATION LIST

### Patent Literature

Patent Literature 1: JP 6464430 B

### SUMMARY

### Technical Problem

There are some cases where particles are included or formed in the medicine to fill such a cylinder. Possible examples of the particles include silicone oil particles, glass particles, plastic particles, and rubber particles, which derive from the syringe; and protein aggregates or powder, which derive from the medicine. In the above cylinder, it is conceivable that these particles are filtered out using a filter, but there can be a risk of increasing discharging resistance at the time of injecting the medicine.

It is an object of the present invention to provide a syringe configured to suppress discharging resistance of a medicine while capturing particles within the medicine at the time of injecting the medicine.

### Solution to Problem

A syringe of the present invention is a syringe for injecting a medicine, the syringe including: a barrel formed into a cylindrical shape with a distal end part and a proximal end part, the distal end part provided with an outlet opening for discharging the medicine contained inside the barrel therethrough; a filter disposed closer to a proximal side than the outlet opening inside the barrel; and a holding member configured to hold the filter disposed inside the barrel, the holding member including a proximal side part disposed closer to the proximal side than the filter, in which the proximal end side part of the holding member has a flow path through which the medicine flows from the proximal side toward a distal side, a proximal side space into which the medicine flows through the flow path is provided on the proximal side of the filter, a distal side space into which the medicine flows from the proximal side space via the filter is provided on the distal side of the filter, and an area of the outlet opening is smaller than an area of the proximal side space, smaller than an area of the distal side space, and smaller than an area of the flow path, as viewed from an axial direction of the barrel.

The syringe can be configured such that the area of the flow path in the holding member is smaller than the area of the proximal side space, as viewed from the axial direction of the barrel.

The syringe can be configured such that the proximal side part of the holding member has, as a proximal end surface, an abutting surface with which a piston inserted from the proximal side into the barrel can come into abutting contact, and the abutting surface is configured to come into surface contact with a distal end surface of the piston over the entire circumference in a circumferential direction of the barrel.

### BRIEF DESCRIPTION OF DRAWINGS

Fig. 1 is a side view of a prefilled syringe provided with a syringe according to this embodiment.
Fig. 2 is a cross-sectional view taken along line II-II in Fig. 1.
Fig. 3 is an enlarged cross-sectional view of an area indicated by III in Fig. 2.
Fig. 4 is a schematic view for explaining the mounting of a filter in the syringe.
Fig. 5 is a side view of a prefilled syringe according to a modified example.
Fig. 6 is an enlarged cross-sectional view of an area indicated by VI in Fig. 5.
Fig. 7 is a side view of a container with a syringe according to a modified example.
Fig. 8 is an enlarged cross-sectional view of an area indicated by VIII in Fig. 7.

### DESCRIPTION OF EMBODIMENTS

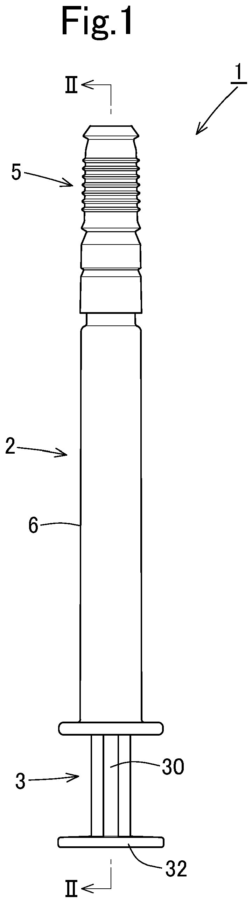

A description will be hereinafter given on a prefilled syringe provided with a syringe according to an embodiment of the present invention, with reference to Fig. 1 to Fig. 4.

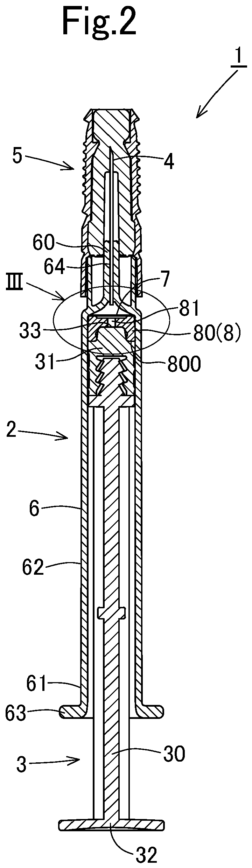

As shown in Fig. 1 and Fig. 2, a prefilled syringe 1 includes a syringe 2 for injecting a medicine. The prefilled syringe 1 further includes a piston 3 inserted into the syringe 2 (see Fig. 2). Furthermore, the prefilled syringe 1 includes an injection needle 4 connected to the syringe 2. The prefilled syringe 1 includes a cap 5 that covers the injection needle 4. The medicine is a liquid medicine, and is, for example, a protein preparation.

The syringe 2 includes a barrel 6 formed into a cylindrical shape having a distal end part 60 and a proximal end part 61, a filter 7 disposed inside the barrel 6, and a holding member 8 configured to hold the filter 7. Further, the syringe 2 has a substantially cylindrical shape. The syringe 2 of this embodiment includes a single filter 7, but can include a plurality of filters 7 layered on each other.

Hereinafter, in the prefilled syringe 1 and the syringe 2, a side on which the distal end part 60 is disposed (upper side in Fig. 1 to Fig. 3) is simply referred to as "distal side", and a side on which the proximal end part 61 is disposed (lower side in Fig. 1 to Fig. 3) is referred to as "proximal side". Also, an axial direction of the syringe 2 is simply referred to as "axial direction".

The barrel 6 is a member for containing a medicine thereinside. The barrel 6 of this embodiment includes, in addition to the distal end part 60 and the proximal end part 61, a cylindrical part 62 connecting the distal end part 60 and the proximal end part 61 to each other (see Fig. 2). Further, the barrel 6 includes a flange part 63 extending outward from the entire outer circumference of the cylindrical part 62 (i.e., extending radially outward of the cylindrical part 62) at an other end in the axial direction of the cylindrical part 62.

The barrel 6 is made of, for example, a material that is transparent and that can withstand the internal pressure applied thereto at the time of injecting the medicine. Specifically, the material of the barrel 6 is a resin having a cyclic olefin such as norbornene in a repeating unit. More specifically, the material of the barrel 6 is a transparent resin such as a COP (cycloolefin polymer) which is a homopolymer of a cyclic olefin, or a COC (cycloolefin copolymer) which is a copolymer of a cyclic olefin and ethylene or the like. The barrel 6 can be made of PP (polypropylene) or glass.

The barrel 6 has silicone oil applied to its inner circumferential surface (e.g., inner circumferential surface of the cylindrical part 62) to suppress sliding resistance of the piston 3 to the inner circumferential surface of the barrel 6.

The distal end part 60 of the barrel 6 is provided at an end in the axial direction of the cylindrical part 62 (specifically, an end part on the distal side). The distal end part 60 is provided with an outlet opening 64 for discharging to the outside the medicine contained inside the barrel 6. In the barrel 6 of this embodiment, the outlet opening 64 is a needle hole through which an injection needle 4 is inserted.

When the needle being inserted through the outlet opening 64 is, for example, a 27-gauge needle, the inner diameter of the outlet opening 64 is 0.27 mm and the area of the outlet opening 64 as viewed from the axial direction is 0.057 mm². When the needle being inserted through the outlet opening 64 is, for example, a 29-gauge needle, the inner diameter of the outlet opening 64 is 0.21 mm and the area of the outlet opening 64 as viewed from the axial direction is 0.035 mm². The outlet opening 64 is substantially constant in cross-section in a direction orthogonal to an axial center of the outlet opening 64.

The distal end part 60 has a proximal end surface 600 formed to close an edge portion on the distal side of the cylindrical part 62 except the outlet opening 64. The proximal end surface 600 extends in an inclined manner to be positioned closer to the distal side as it advances radially inward.

The area of the cylindrical part 62 as viewed from the axial direction (i.e., the cross-sectional area of the cylindrical part 62 in the direction orthogonal to the axial center) is for example substantially constant, and specifically 12.56 mm² or more and 314 mm² or less. The inner diameter of the cylindrical portion 62 is for example substantially constant, and specifically 4 mm or more and 20 mm or less.

The holding member 8 is a member to which the filter 7 is attached. The holding member 8 is housed in the barrel 6 in a state of being in pressure contact with the barrel 6. Specifically, at least a part of an outer circumferential surface of the holding member 8 is in close contact with the inner circumferential surface of the barrel 6.

The holding member 8 is formed of a flexible material. The holding member 8 of this embodiment is more flexible than the barrel 6. Specifically, the material of the holding member 8 is, for example, a resin, a rubber, an elastomer, or the like, which is softer than the barrel 6. The flexibility of the barrel 6 and the holding member 8 can be confirmed by, for example, measuring their durometer hardnesses. The holding member 8 formed of such a material can ensure its flexibility relative to the barrel 6 to prevent cracks of the barrel 6.

The holding member 8 includes a proximal side part 80 disposed closer to the proximal side than the filter 7. The holding member 8 of this embodiment is composed only of the proximal side part 80. The proximal side part 80 of the holding member 8 is provided with a flow path 81 through which the medicine flows from the proximal side toward the distal side.

The proximal side part 80 of the holding member 8 has, as a proximal end surface 800, an abutting surface 800 with which the piston 3 to be inserted into the barrel 6 from the proximal side can come into abutting contact. The proximal side part 80 has a circular cylindrical shape with its distal side closed with a disk having a through hole at the center.

The abutting surface 800 of the proximal side part 80 has a shape corresponding to a distal end surface 33 of the piston 3. The abutting surface 800 is formed to come into surface contact with the distal end surface 33 of the piston 3 over the entire circumference in the circumferential direction of the barrel 6.

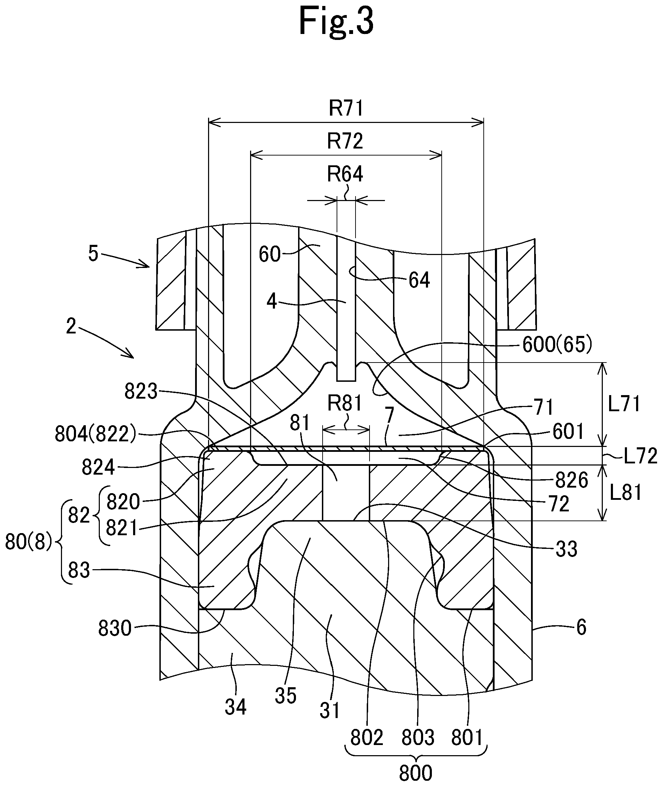

As shown in, for example, Fig. 3, the abutting surface 800 of this embodiment includes a proximal side abutting surface 801 located on the proximal side, a distal side abutting surface 802 located on the distal side, and a connecting abutting surface 803 connecting the proximal side abutting surface 801 and the distal side abutting surface 802 to each other.

The proximal side abutting surface 801 is, for example, a substantially perpendicular surface to the axial direction. The distal side abutting surface 802 is, for example, a substantially perpendicular surface to the axial direction.

The connecting abutting surface 803 is a surface extending along the axial direction. For example, the central portion in the axial direction of the connecting abutting surface 803 is recessed radially outward.

The proximal side part 80 of this embodiment includes an attachment portion 82 to which the filter 7 is attached, and a leg portion 83 extending from the attachment portion 82. In this proximal side part 80, the attachment portion 82 is provided on the distal side, and the leg portion 83 is disposed on the proximal side.

The flow path 81 is provided through the attachment portion 82 of the proximal side part 80. The area of the flow path 81 of this embodiment as viewed from the axial direction (i.e., cross-sectional area of the flow-path 81 in the direction orthogonal to the axial center) is constant. The area of the flow path 81 as viewed from the axial direction is 0.64 mm² or more and 1.33 mm² or less. The internal diameter of the flow path 81 of this embodiment is constant. The inner diameter of the flow path 81 is, for example, 0.3 mm or more and 0.9 mm or less. The flow path 81 is a through hole and specifically has a circular cylindrical shape, but can have a polygonal cylindrical shape.

The attachment portion 82 has such a shape as to have its outer circumferential part projecting toward the distal side. The outer circumferential part of the attachment portion 82 of this embodiment is located slightly away from the inner circumferential surface of the barrel 6 (specifically, the inner circumferential surface of the cylindrical part 62 of the barrel 6). Further, the attachment portion 82 has a tapered shape having a smaller outer diameter as it advances to the distal side. The attachment portion 82 includes an attachment outer circumferential part 820 forming the outer circumferential part of the attachment portion 82, and an attachment inner circumferential part 821 located radially inside the attachment outer circumferential part 820 and defining the flow path 81.

Furthermore, in the attachment portion 82, an outer circumferential distal end surface 822, which is a distal end surface of the attachment outer circumferential part 820, is located closer to the distal side than an inner circumferential distal end surface 823, which is a distal end surface of the attachment inner circumferential surface 821. In other words, the distal end surface of the attachment portion 82 is recessed at its central portion in the circumferential direction. The attachment outer circumferential part 820 includes a shoulder part 824 as an outer circumferential part on the distal side.

The leg portion 83 has such a shape as to have its outer circumferential part projecting toward the proximal side. The proximal end surface 830 of the leg portion 83 forms the abutting surface 800 of the proximal side part 80. The outer circumferential surface of the leg portion 83 is in surface contact with the inner circumferential surface of the barrel 6 (specifically, the inner circumferential surface of the cylindrical part 62 of the barrel 6). The configuration that the outer circumferential surface of the leg portion 83 is tightly attached to the inner circumferential surface of the barrel 6 allows the medicine to securely flow through the flow path 81 of the holding member 8 and be filtered by the filter 7.

The filter 7 is a filter for filtering the medicine. The filter 7 is disposed closer to the proximal side than the outlet opening 64 inside the barrel 6.

A proximal side space 72 into which the medicine flows through the flow path 81 flows is provided on the proximal side of the filter 7. A distal side space 71 into which the medicine flows from the proximal side pace 72 via the filter 7 is provided on the distal side of the filter 7. The filtration area of the filter 7 corresponds to the area defined by an end edge on the distal side of the proximal side space 72, and to the area defined by an end edge on the proximal side of the distal side space 71. The area defined by the end edge on the distal side of the proximal side space 72 is, for example, equal to the area defined by the end edge on the proximal side of the distal side space 71.

The filter 7 can be made of, for example, a membrane, a mesh, a sintered body, or a foam body. The material of the filter 7 is a resin, a ceramic, a metal, paper, or the like.

The filter 7 is, for example, a membrane filter. The filter 7 can be a prefilter.

The filter 7 of this embodiment is attached to a distal end surface 804 of the proximal side part 80 of the holding member 8. Specifically, the filter 7 is welded to the distal end surface 804. More specifically, the filter 7 is welded to the outer circumferential distal end surface 822 of the attachment outer circumferential part 820. This welding is ultrasonic welding, but a different welding method such as heat welding can be used. The filter 7 can adhere to the distal end surface 804.

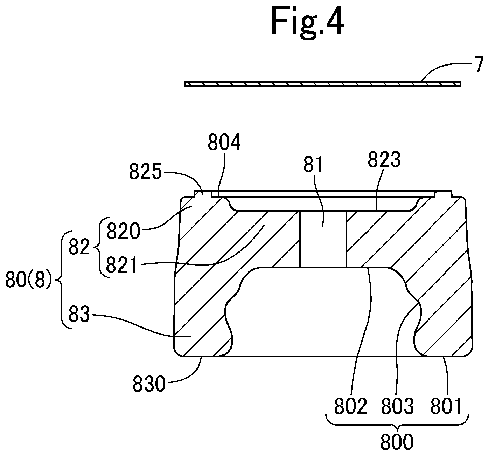

As shown in Fig. 4, the proximal side part 80 (e.g., attachment outer circumferential part 820) is provided with a projecting part 825 projecting toward the distal side in the state before the filter 7 is welded. When the filter 7 is welded, the projecting part 825 melts and spreads to make substantially flat the distal end surface 804 of the proximal side part 80 in the state after the filter 7 is welded.

The distal side space 71 is defined by the inner circumferential surface 65 of the barrel 6 (specifically, the proximal side end surface 600 of the distal end part 60) and the filter 7 (specifically, the distal side surface of the filter 7). The distal side space 71 is continuous with the outlet opening 64.

Further, the distal side space 71 has such a shape as to have a smaller area when viewed from a cylindrical direction (i.e., a smaller cross-sectional area in the direction orthogonal to the axial center) as it advances from the proximal side toward the distal side, that is, has a tapered shape having a smaller area when viewed from the cylindrical direction (i.e., a smaller cross-sectional area in the direction orthogonal to the axial center) as it advances toward the distal side. The distal side space 71 of this embodiment has a substantially circular cylindrical shape. This distal side space 71 has, for example, such a shape as to have a smaller inner diameter as it advances from the proximal side toward the distal side, that is, has a tapered shape having a smaller inner diameter as it advances toward the distal side. The distal side space 71 has such a shape as to have a smaller distance between the filter 7 and the proximal side end surface 600 of the distal end part 60 of the barrel 6 as it advances radially outward. This configuration allows the medical liquid to easily move in the circumferential direction from the outer circumferential side to a cylindrical shaft side to be pressed into the distal side space 71 at the time of injecting the medicine, thereby being capable of suppressing discharging resistance. The distal side space 71 can have a polygonal cylindrical shape.

A proximal side portion of the distal side space 71, that is, a portion of the distal side space 71 having the largest area (e.g., inner diameter) is lined with the shoulder part 824 in the axial direction of the barrel 6, and has an inner diameter substantially the same as the outer diameter of the shoulder part of the holding member 8.

The proximal side space 72 is defined by the filter 7 (specifically, the proximal side surface of the filter 7) and the distal end surface 804 of the proximal side part 80 (specifically, the inner circumferential distal end surface 823 of the attachment inner circumferential part 821 and an attachment inner circumferential surface 826 which is the inner circumferential surface of the attachment outer circumferential part 820). The proximal side space 72 is formed with the inner circumferential distal end surface 823 of the attachment inner circumferential part 821 recessed toward the proximal side from the outer circumferential distal end surface 822 of the attachment outer circumferential part 820, that is, formed with the distal end surface of the attachment portion 82 recessed toward the proximal side.

The proximal side space 72 is continuous with the flow path 81. The proximal side space 72 has, for example, such a shape as to have a larger area when viewed from the axial direction as it advances from the proximal side toward the distal side, that is, has a tapered shape having a larger area when viewed from the axial direction as it advances toward the distal side. The proximal side space 72 of this embodiment has a substantially circular cylindrical shape. This proximal side space 72 has, for example, such a shape as to have a larger inner diameter as it advances from the proximal side toward the distal side, that is, has a tapered shape having a larger inner diameter as it advances toward the distal side. The inclination angle of the attachment inner circumferential surface 826 of the attachment outer circumferential part 820 defining the proximal side space 72 with respect to the axial direction is smaller than the inclination angle of the proximal end surface 600 of the distal end part 60 defining the distal side space 71. The proximal side space 72 can have a polygonal cylindrical shape.

In the syringe 2 as described above, the barrel 6 and the proximal side part 80 of the holding member 8 have the filter 7 sandwiched therebetween. Specifically, the filter 7 is held sandwiched in the axial direction between the proximal side end surface 600 of the distal end part 60 of the barrel 6 (specifically, the proximal side end edge 601 of the proximal end surface 600) and the shoulder part 824 of the proximal side part 80. This configuration allows the filter 7 to be hardly detached from the proximal side part 80 at the time of injecting the medicine even when the filter 7 is subjected to pressure.

The flow path 81, the proximal side space 72, the distal side space 71, and the outlet opening 64 are sequentially arranged in this order from the proximal side toward the distal side. In the syringe 2 of this embodiment, the central axis of the flow path 81, the central axis of the proximal side space 72, and the central axis of the distal side space 71 all coincide with the central axis of the outlet opening 64. This configuration allows the medicine to favorably flow from the flow path 81 to the outlet opening 64 at the time of injecting the medicine.

The dimension L81 in the axial direction of the flow path 81 is larger than the dimension L72 in the axial direction of the proximal side space 72. The dimension L71 in the axial direction of the distal side space 71 (specifically, the dimension of the central axis of the distal side space 71) is larger than the dimension L72 in the axial direction of the proximal side space 72.

When viewed from the axial direction, the area of the outlet opening 64 is smaller than the area of the proximal side space 72, smaller than the area of the distal side space 71, and smaller than the area of the flow path 81. This means that the area of the outlet opening 64 is smaller than the area of any portion in the axial direction of the proximal side space 72, smaller than the area of any portion in the axial direction of the distal side space 71, and is smaller than the area of any portion in the axial direction of the flow path 81. Specifically, the area of the outlet opening 64 is smaller than the area of the proximal side space 72, smaller than the maximum area of the distal side space 71 (i.e., area defined by the proximal side end edge of the distal side space 71), and smaller than the area of the flow path 81.

When viewed from the axial direction, the area of the flow path 81 is smaller than the area of the proximal side space 72. This means that the area of the flow path 81 is smaller than the area of any portion in the axial direction of the proximal side space 72.

Specifically, the inner diameter R64 of the outlet opening 64 is smaller than the inner diameter R72 of the proximal side space 72, smaller than the maximum inner diameter R71 of the distal side space 71 (i.e., the inner diameter at the proximal end edge of the distal side space 71), and smaller than the inner diameter R81 of the flow path 81. The inner diameter R81 of the flow path 81 is smaller than the inner diameter R72 of the proximal side space 72.

An experiment was performed for these dimensional relationships by combining the barrel 6 with the inner diameter R64 of the outlet opening 64 of 0.27 mm or 0.21 mm and the holding member 8 with the inner diameter R81 of the flow path 81 of 0.3 mm, 0.6 mm, or 0.9 mm and with the inner diameter R72 of the proximal side space 72 of 0.6 mm, 2.1 mm, or 3.6 mm to examine an effect on discharging resistance (specifically, sliding load).

The results of this experiment are as follows. When the inner diameter R72 of the proximal side space 72 was 0.6 mm, an increase in sliding load was confirmed. When the inner diameter R72 of the proximal side space 72 was 2.1 m or 3.6 mm, no significant change in sliding load was observed. Further, when the inner diameter R81 of the flow path 81 was caused to change within the above range, no significant change in sliding load was observed.

For example, the inner diameter R81 of the flow path 81 is preferably more than 1 time and 5 times or less as large as the inner diameter R64 or the outlet opening 64, and is desirably 1.1 times or more and 4.3 times or less as large as the inner diameter 64 of the outlet opening 64. When the inner diameter R64 of the outlet opening 64 is 0.21 mm or 0.27 mm, the inner diameter R81 of the flow path 81 can be selected from any one of 0.3 mm, 0.6 mm, and 0.9 mm. For example, the inner diameter R72 of the proximal side space 72 (the filtration area of the filter 7) is preferably 5 times or more and 20 times or less as large as the inner diameter R64 of the outlet opening 64, and is desirably 7 times or more and 18 times or less as large as the inner diameter R64 of the outlet opening 64. When the inner diameter R64 of the outlet opening 64 is 0.21 mm or 0.27 mm, the inner diameter R72 of the proximal side space 72 can be selected from any one of 2.1 mm and 3.6 mm. The configuration that the inner diameters R72 and R71 respectively of the proximal side space 72 and the distal side space 71 fall within these respective ranges can suppress discharging resistance while ensuring the filtration area at the time of injecting the medicine.

The syringe 2 of this embodiment is sterilized by radiation such as γ-rays. This sterilization can be performed by a gas such as ethylene oxide gas, or by autoclaving.

The piston 3 is a member operated when the medicine inside the barrel 6 is discharged. The piston 3 of this embodiment includes: a rod part 30 having a shaft shape; a gasket 31 attached to one end in a longitudinal direction of the rod part 30 and configured to be brought into close contact with the entire inner circumference of the cylindrical part 62 of the barrel 6; and an operating part 32 attached to the other end in the longitudinal direction of the rod part 30.

In the piston 3 of this embodiment, the distal end surface 33 is formed of a distal end surface of the gasket 31. Specifically, at the time of injecting the medicine, the distal end surface 33 is configured to be in abutting contact at least with the proximal side abutting surface 801 of the proximal side part 80 of the holding member 8, more specifically, configured to be in abutting contact with the connecting abutting surface 803 in addition to the proximal side abutting surface 801.

The piston 3 further includes a close contact part 34 configured to be brought into close contact with the inner circumferential surface of the barrel 6, and a projecting part 35 projecting from a distal end surface of the close contact part 34. The configuration is such that the distal end surface of the close contact part 34 comes into abutting contact with the proximal side abutting surface 801, and the distal end surface of the projecting part 35 comes into close contact with the distal side abutting surface 802. An outer circumferential surface of the projecting part 35 is configured to come into close contact with the connecting abutting surface 803.

The injection needle 4 is a member for injecting the medicine inside the barrel 6 to a patient. The tip of the injection needle 4 is covered by a cap 5.

The syringe 2 configured as above allows the filter 7 to capture particles even when they are present in the medicine contained in the barrel 6 at the time of injecting the medicine, and further allows the medicine to flow through the flow path 81, the proximal side space 72, and the distal side space 71, each of which has a larger area (e.g., inner diameter) than that of the outlet opening 64, to be discharged through the outlet opening 64, thereby being capable of suppressing resistance when the medicine flows through the flow path 81, the proximal side space 72, and the distal side space 71. As a result, the discharging resistance of the medicine can be suppressed.

The syringe 2 of this embodiment, which includes the flow path 81 having a small area (e.g., inner diameter) and the proximal side space 72 having a large area (e.g., inner diameter), can reduce the amount of medicine remaining in the flow path 81 while ensuring the area of the filter 7 with which the medicine is to be in contact (i.e., filtration area of the filter 7), thereby suppressing the amount of residual liquid while suppressing discharging resistance.

The syringe 2 of this embodiment is configured to have the distal end surface 33 of the piston 3 moving until it comes into surface contact with the abutting surface 800 of the holding member 8 over the entire circumference in the circumferential direction at the time of injecting the medicine, thereby being capable of suppressing the amount of the residual liquid between the piston 3 and the holding member 8 and capable of suppressing the amount of residual liquid within the barrel 6.

It is a matter of course that the syringe of the present invention is not limited to the aforementioned embodiment, but various modifications can be made without departing from the gist of the present invention. For example, a configuration of an embodiment can be added to a configuration of another embodiment, and part of a configuration of an embodiment can be replaced by a configuration of another embodiment. Further, part of a configuration of an embodiment can be deleted.

The configuration of the holding member 8 can differ from the above configuration. For example, the abutting surface 800 of the proximal end portion 80 can have a different shape as long as it has such a shape as to conform to the abutting surface 33 of the piston 3.

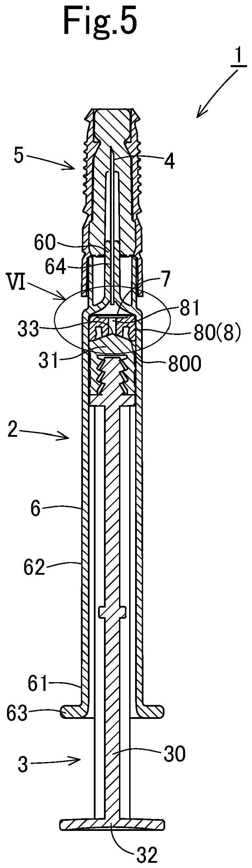

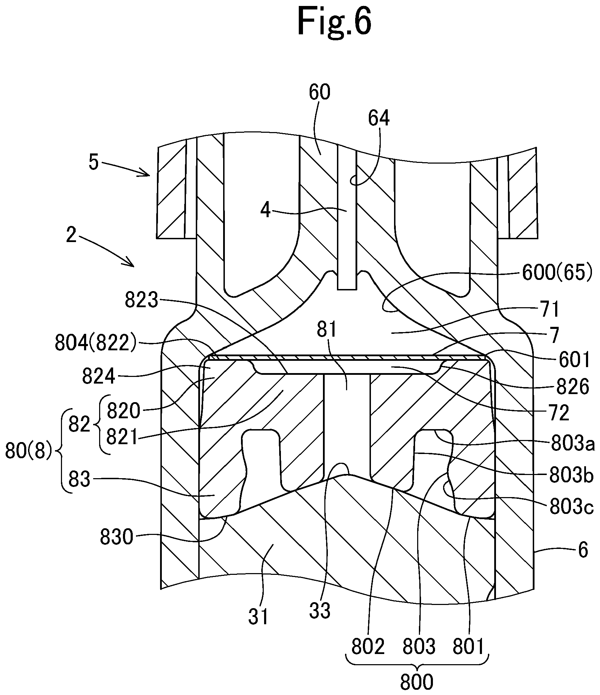

Specifically, the connecting abutting surface 803 extends along the axial direction in the aforementioned embodiment, but can include a portion extending in a direction inclined relative to the axial direction. More specifically, as shown in Fig. 5 and Fig. 6, the configuration can be such that the connecting abutting surface 803 includes a first connecting abutting surface 803a, which is a surface substantially perpendicular to the axial direction, and second connecting abutting surfaces 803b, 803c, which are a pair of surfaces extending along the axial direction from the both ends of the first connecting abutting surface 803a.

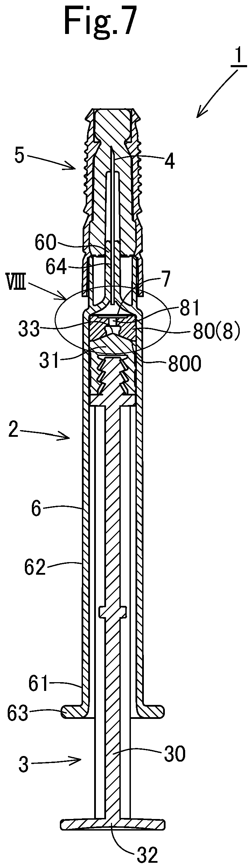

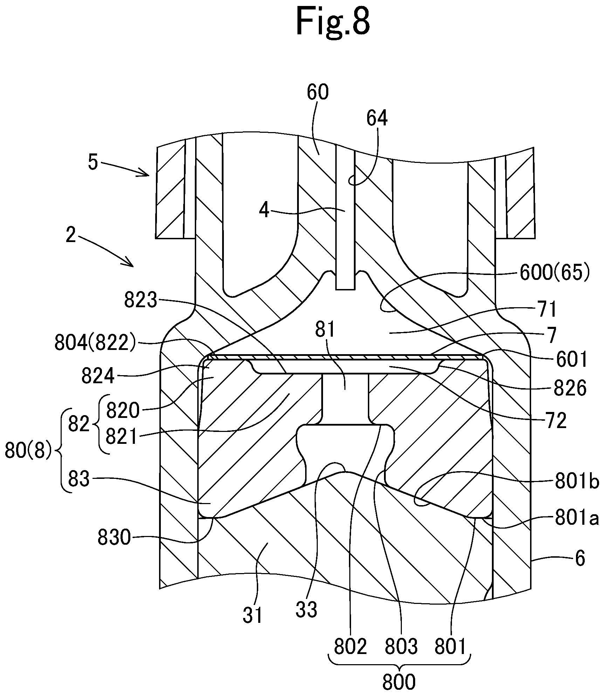

Specifically, the proximal side abutting surface 801 is a surface substantially perpendicular to the axial direction in the aforementioned embodiment, but can include a portion included at an angle other than at a right angle relative to the axial direction. More specifically, as shown in Fig. 7 and Fig. 8, the configuration can be such that the proximal side abutting surface 801 includes a first proximal side abutting surface 801a that is a substantially perpendicular surface to the axial direction, and a second proximal side abutting surface 801b that further extends radially inward from a radially inside end edge of the first proximal side abutting surface 801a and is inclined to be positioned on the distal side as it advances radially inward.

Even with such a configuration, the distal end surface 33 of the piston 3 moves until it comes into surface contact with the abutting surface 800 of the holding member 8 over the entire circumference in the circumferential direction to be thereby capable of suppressing the amount of the residual liquid between the piston 3 and the holding member 8 and capable of suppressing the amount of the residual liquid within the barrel 6.

The holding member 8 is formed of the proximal side part 80 in the aforementioned embodiment, but can include a distal side part that is disposed closer to the distal side than the filter 7 and is formed integrally with or separately from the proximal side part 80.

In the aforementioned embodiment, the flow path 81 in the holding member 8 has an inner diameter smaller than the inner diameter of the proximal side space 72, but the inner diameter of the flow path 81 can substantially coincide with the inner diameter of the proximal side space 72.

In the aforementioned embodiment, the abutting surface 800 of the proximal side part 80 of the holding member 8 is configured to come into surface contact with the distal end surface 33 of the piston 3 over the entire circumference in the circumferential direction of the barrel 6, but the abutting surface 800 can be configured to come into surface contact with the distal end surface 33 of the piston 3 partially in the circumferential direction. For example, it is conceivable that the proximal side abutting member 801 of the abutting surface 800 of the proximal side part 80 is provided with recesses or projections intermittently in the circumferential direction.

In the aforementioned embodiment, the distal side space 71 in the syringe 2 has a tapered shape having a smaller inner diameter as it advances toward the distal side, but the distal side space 71 can have a shape having a uniform inner diameter or other shapes. In this case, it is conceivable that the proximal end surface 600 extends, for example, in a direction perpendicular to the axial direction.

The proximal side space 72 has a tapered shape having a smaller inner diameter as it advances toward the distal side, but the proximal side space 72 can have a shape having a uniform inner diameter or other shapes.

The dimension in the axial direction of the flow path 81 can be equal to or less than the dimension in the axial direction of the proximal side space 72.

Further, the dimension in the axial direction of the distal side space 71 can be equal to or less than the dimension in the axial direction of the proximal side space 72. In this case, for example, it is conceivable that the proximal side part 80 of the holding member 8 is disposed at a position at which it is in abutting contact with the proximal end surface 600 of the distal end portion 60 of the barrel 6 or is made to have a large dimension in the axial direction while the inclination angle of the proximal end surface 600 is made small with respect to the cylinder shaft.

According to the present invention, provided can be a syringe configured to suppress discharging resistance of a medicine while capturing particles within the medicine at the time of injecting the medicine.

A syringe of the present invention is a syringe for injecting a medicine, the syringe including: a barrel formed into a cylindrical shape with a distal end part and a proximal end part, the distal end part provided with an outlet opening for discharging to the outside the medicine contained inside the barrel therethrough; a filter disposed closer to a proximal side than the outlet opening inside the barrel; and a holding member configured to hold the filter disposed inside the barrel, the holding member including a proximal side part disposed closer to the proximal side than the filter, in which the proximal side part of the holding member has a flow path through which the medicine flows from the proximal side toward a distal side, a proximal side space into which the medicine flows through the flow path is provided on the proximal side of the filter, a distal side space into which the medicine flows from the proximal side space via the filter is provided on the distal side of the filter, and an area of the outlet opening is smaller than an area of the proximal side space, smaller than an area of the distal side space, and smaller than an area of the flow path, as viewed from an axial direction of the barrel.

Such a configuration allows the filter to capture particles even when they are present in the medicine contained in the barrel at the time of injecting the medicine, and further allows the medicine to flow through the flow path, the proximal side space, and the distal side space, each of which has a larger area than that of the outlet opening, to be discharged through the outlet opening, thereby being capable of suppressing resistance when the medicine flows through the flow path, the proximal side space, and the distal side space. As a result, the discharging resistance of the medicine can be suppressed.

The syringe can be configured such that the area of the flow path in the holding member is smaller than the area of the proximal side space, as viewed from the axial direction of the barrel.

Such a configuration in which the area of the flow path is made small and the area of the proximal side space is made large can reduce the amount of medicine remaining in the flow path while ensuring the area of the filter with which the medicine is to be in contact (i.e., filtration area of the filter), thereby suppressing the amount of residual liquid while suppressing discharging resistance.

The syringe can be configured such that the proximal side part of the holding member has, as a proximal end surface, an abutting surface with which a piston inserted from the proximal side into the barrel can come into abutting contact, and the abutting surface is configured to come into surface contact with a distal end surface of the piston over the entire circumference in a circumferential direction of the barrel.

According to such a configuration, the distal end surface of the piston moves until it comes into surface contact with the abutting surface of the holding member over the entire circumference in the circumferential direction at the time of injecting the medicine, thereby being capable of suppressing the amount of the residual liquid between the piston and the holding member and capable of suppressing the amount of the residual liquid within the barrel.

### REFERENCE SIGNS LIST

1: Container
2: Syringe
3: Piston
4: Injection needle
5: Cap
6: Barrel
7: Filter
8: Holding member
30: Rod part
31: Gasket
32: Operating part
33: Abutting surface (distal end surface)
34: Close contact part
35: Projecting part
60: Distal end part
61: Proximal end part
62: Cylindrical part
63: Flange part
64: Outlet opening
65: Inner circumferential surface
71: Distal side space
72: Proximal side space
80: Proximal side part
81: Flow path
82: Attachment portion
83: Leg portion
600: Proximal end surface
601: Proximal end edge
800: Abutting surface (proximal end surface)
801: Proximal side abutting surface
801a: First proximal side abutting surface
801b: Second proximal side abutting surface
802: Distal side abutting surface
803: Connecting abutting surface
803a: First connecting abutting surface
803b: Second connecting abutting surface
803c: Second connecting abutting surface
804: Distal end surface
820: Attachment outer circumferential part
821: Attachment inner circumferential part
822: Outer circumferential distal end surface
823: Inner circumferential distal end surface
824: Shoulder part
825: Projecting part
826: Attachment inner circumferential surface
830: Proximal end surface

## Claims

1. A syringe for injecting a medicine, the syringe comprising:
a barrel formed into a cylindrical shape with a distal end part and a proximal end part, the distal end part provided with an outlet opening for discharging to the outside the medicine contained inside the barrel therethrough;
a filter disposed closer to a proximal side than the outlet opening inside the barrel; and
a holding member configured to hold the filter disposed inside the barrel, the holding member comprising a proximal side part disposed closer to the proximal side than the filter, wherein
the proximal side part of the holding member has a flow path through which the medicine flows from the proximal side toward a distal side,
a proximal side space into which the medicine flows through the flow path is provided on the proximal side of the filter,
a distal side space into which the medicine flows from the proximal side space via the filter is provided on the distal side of the filter, and
an area of the outlet opening is smaller than an area of the proximal side space, smaller than an area of the distal side space, and smaller than an area of the flow path, as viewed from an axial direction of the barrel.

2. The syringe according to claim 1, wherein the area of the flow path in the holding member is smaller than the area of the proximal side space, as viewed from the axial direction of the barrel.

3. The syringe according to claim 1 or 2, wherein the proximal side part of the holding member has, as a proximal end surface, an abutting surface with which a piston inserted from the proximal side into the barrel can come into abutting contact, and the abutting surface is configured to come into surface contact with a distal end surface of the piston over the entire circumference in a circumferential direction of the barrel.
